Europäisches Patentamt

**European Patent Office**

Office européen des brevets

(11) Veröffentlichungsnummer: **0 060 832**
**B1**

(12) **EUROPÄISCHE PATENTSCHRIFT**

(45) Veröffentlichungstag der Patentschrift:
**20.03.85**

(21) Anmeldenummer: **80901927.6**

(22) Anmeldetag: **01.10.80**

(86) Internationale Anmeldenummer:
**PCT/DE 80/00141**

(87) Internationale Veröffentlichungsnummer:
**WO 82/01127 (15.04.82** Gazette **82/10)**

(51) Int. Cl.⁴: **A 61 F 2/18**

(54) **GEHÖRKNÖCHELCHEN-PROTHESE.**

(30) Priorität: **26.09.80 DE 3036245**

(43) Veröffentlichungstag der Anmeldung:
**29.09.82 Patentblatt 82/39**

(45) Bekanntmachung des Hinweises auf die Patenterteilung:
**20.03.85 Patentblatt 85/12**

(84) Benannte Vertragsstaaten:
**AT CH DE FR GB LI**

(56) Entgegenhaltungen:
**DE - A - 2 905 183**
**GB - A - 2 041 759**
**US - A - 3 909 852**

(73) Patentinhaber: **ERNST LEITZ WETZLAR GMBH,**
**Ernst-Leitz-Strasse 30 Postfach 20 20,**
**D-6330 Wetzlar 1 (DE)**

(72) Erfinder: **RECK, Ralf, Südring 189,**
**D-6500 Mainz-Bretzenheim (DE)**
Erfinder: **BRÖMER, Heinz, Am Hundsrück 7,**
**D-6331 Hermannstein (DE)**
Erfinder: **DEUTSCHER, Klaus-Konrad, Lerchenweg 20,**
**D-6330 Wetzlar (DE)**

(74) Vertreter: **Lüssem, Heribert, c/o Ernst Leitz Wetzlar**
**GmbH Postfach 2020, D-6330 Wetzlar 1 (DE)**

## Beschreibung

Die Anmeldung betrifft eine aus bioaktivem Material bestehende Gehörknöchelchen-Prothese.

Zur vollständigen oder teilweisen Rekonstruktion der Gehörknöchelchen-Kette werden Implantate aus verschiedenen Materialien verwendet. In letzter Zeit haben Kunststoffe wegen ihres geringen spezifischen Gewichtes und wegen ihrer leichten Bearbeitbarkeit auch in der intraoperativen Phase bei der Anpassung der industriell vorgeformten Implantate an die individuellen Gegebenheiten eine gewisse Bedeutung erlangt. Trotz der geringen Reaktionsfähigkeit des Implantatlagers und der Inertheit der Kunststoffe ist der Langzeiterfolg dieser Implantate im Mittelohr nicht gewährleistet, da sie mit der Zeit das Trommelfell penetrieren.

Aus der DE-A-2 905 183 ist eine einstückige Gehörknöchelchen-Prothese aus bioinertem Al$_2$O$_3$-Material bekannt, die aus einer kreisförmigen Planparallel-Platte mit zentro-symmetrischem Gerad-Schaft oder aber aus einem exzentrisch an der Plattenunterseite in deren Peripherbereich angreifenden Schief-Schaft mit einem Neigungswinkel von ca. 30° gegenüber der Platten-Normalen besteht. Diese bekannte Prothese weist mindestens eine Rille auf der Plattenoberseite auf, wobei in Falle des Vorhandensein nur einer Rille dieselbe Plattenoberfläche zentrosymmetrisch durchquert und im Falle des Vorhandenseins zweier bzw. dreier paralleler Rillen dieselben immer spiegelsymmetrisch auf der kreisförmigen Plattenoberseite angeordnet sind. Es ist ein essentielles Merkmal in der DE-A-2 905 183, dass die Rille bzw. die Rillen bei der Schief-Schaft-Prothese parallel zu der Ebene verlaufen, die durch die Plattennormale und die Schaft-Achse aufgespannt wird.

Das benutzte bioinerte Aluminiumoxid-Material löst im knöchernen Gewebe zwar auch nur geringe Reaktionen aus — wobei die Dicke der sich bildenden, trennenden Bindegewebsschicht als Mass für die Biokompatibilität betrachtet werden kann —; da das Al$_2$O$_3$-Material aber völlig inert im Mittelohrbereich liegt, sind auch hinsichtlich des Langzeiterfolges ähnliche Einschränkungen zu machen wie bei den o.a. Kunststoffen.

Aus dem «Archiv für Ohren-, Nasen- und Kehlkopfheilkunde, Archives of Oto-Rhino-Laryngology», Band 223, Heft 2-4, 1979, Seiten 369-372, sind darüber hinaus aus bioaktiver Glaskeramik bestehende Tympanoplastiken bekannt geworden, die in vivo von einer bis 40 $\mu$m dicken Knochenschicht umgeben werden. Die Form der dargestellten Prothesen entspricht einer exzentrischen Gerad-Schaft-Anordnung, wobei die Plattenoberseite aus einer nicht-planen, mugeligen bzw. ellipsoidförmigen Fläche besteht und keine rillenförmige Einkerbung aufweist.

Bei den bisher bekannten Implantatformen hat es sich als nachteilig herausgestellt, dass sie nicht in optimaler Weise der komplizierten Mittelohr-Anatomie anpassbar sind und dass sie wegen der speziellen lokalen Gegebenheiten nicht langzeitstabil gehalten werden können. Bei einer Rillenausrichtung parallel zur Symmetrieebene einer aus der DE-A-2 905 183 bekannten Schief-Schaft-Prothese besteht auf Dauer die Gefahr, dass sich bei einem Patienten, der unvorhergesehenen starken Erschütterungsimpulsen insbesondere in Richtung der Vertikalen des menschlichen Körpers — wie sie etwa bereits bei Sprüngen, beim Treppensteigen oder erst recht bei Unfällen usw. ausgelöst werden können — ausgesetzt ist, die implantierte Prothese aus ihrer rein mechanischen Verankerung «geschüttelt» wird.

Des weiteren hat es sich zwischenzeitlich bei vielen Anwendungsfällen als nachteilig erwiesen, dass bei bekannten Prothesen-Formen die Plattenoberfläche kugelkalottenförmig ausgebildet ist, da dies zu einer ungleichmässigen und lokal stark überhöhten Druckausübung bei Kontaktierung mit dem Trommelfell führt. Darüber hinaus bietet eine etwa nachträglich eingefräste Rille, insbesondere dann, wenn diese nicht zentrosymmetrisch auf einer linsenkopfförmigen Plattenoberseite eingelassen wird, nur einen unverhältnismässig geringen mechanischen Halt für das in die Rille eingreifende körpereigene Gehörgang-Fragment.

Der Erfindung liegt daher die Aufgabe zugrunde, lokalanatomisch optimal angepasste bzw. anpassbare Gehörknöchelchen-Prothesen anzugeben, an welchen — soweit es ihre Raumform betrifft — im individuellen Bedarfsfall gegebenenfalls lediglich geringfügige mechanische Veränderungen vorgenommen werden müssen, beispielsweise eine Schaftlängenkorrektur, bzw. welche — soweit es das Prothesenmaterial betrifft — aufgrund ihres besonderen Behandlungs-Finish einen mehrere Zell-Lagen dicken Lamellenmantel auf der Prothesenoberfläche in vivo entstehen lässt.

Die Aufgabe wird bei einer Gehörknöchelchen-Prothese, enthaltend eine Platte mit einer Plattenoberseite und einer Plattenunterseite, wobei die Platte eine spiegelsymmetrische Form bezüglich einer auf den durch die Plattenoberseite und durch die Plattenunterseite definierten Ebenen senkrecht stehenden Symmetrieebene aufweist, einen an der Plattenunterseite derart exzentrisch angesetzten Schaft, dass der geometrische Mittelpunkt von dessen Ansatzfläche auf der Spur dieser Symmetrieebene, jedoch mindestens um den entlang der Spur gemessenen Halbmesser der Ansatzfläche vom Rand der Platte entfernt liegt, und eine die Plattenoberseite durchlaufende Rille, erfindungsgemäss dadurch gelöst, dass die einzige Rille auf der den geometrischen Mittelpunkt der Ansatzfläche nicht enthaltenden Hälfte der Platte angeordnet und senkrecht zu der Symmetrieebene ausgerichtet ist und die Prothese aus bioaktivem Material besteht.

Es ist zweckmässig, dass die Platte die Form eines Rechteckes hat und so angeordnet ist, dass die längere Rechteckseite parallel zur Spur der Symmetrieebene der Prothese verläuft. Auch ist es möglich, dass die Platte die Form einer Ellipse hat und so angeordnet ist, dass die längere Ellipsenachse mit der Spur der Symmetrieebene der Prothese zusammenfällt.

Weiterhin kann die Ausbildung der Platte so getroffen sein, dass sie lediglich eine auf ihr senkrecht stehende Symmetrieebene aufweist und diese mit der Spur der Symmetrieebene der Prothese zusammenfällt. Die Kanten der Plattenoberseite und die Ecken der Platten können vorteilhafterweise abgerundet sein. Auch ist es möglich, dass die Dicke der Platte zu ihrer vorderen Hälfte hin entweder stetig ab- oder zunimmt. Nach einer bevorzugten Ausführungsform schliesst die Achse des Schaftes mit der Normalen der Plattenunterseite einen Winkel $\alpha$ in der Grössenordnung von $\alpha = 15° \pm 5°$ ein. Es kann zweckmässig sein, den Querschnitt des Schaftes entweder kreis- oder ellipsenförmig auszubilden. Von Vorteil kann eine Schaftform sein, die sich — ausgehend von der Ansatzfläche des Schaftes an der Plattenunterseite — zum Schaftende hin stetig verjüngt. Zweckmässigerweise weist der Querschnitt der Rille die Form eines Halbkreises oder eines Dreiecks mit abgerundeter Spitze auf. Es ist auch möglich, dass die Kanten, die die Rillen mit der Plattenoberseite bildet, nichtparallel verlaufen.

Auch kann die Ausbildung der Rille in der Plattenoberseite so getroffen sein, dass die Kanten, die die Rille mit der Plattenoberseite bildet, abgerundet sind. Vorteilhafterweise entspricht die Tiefe der Rille maximal der halben Plattendicke an deren Vorderkante und der Abstand zwischen der Vorderkante der Plattenoberseite und der ersten Kante der Rille mindestens der halben Breite der Rille.

Nach einer bevorzugten Ausbildungsform besteht die Prothese aus einem bioaktiven Verbundmaterial, beispielsweise einem solchen auf der Basis von Methylmetacrylat mit dispers eingelagerten bioaktiven Partikeln, oder einem apatithaltigen Sinterprodukt, oder einer bioaktiven Glaskeramik, oder einem Bio-Glas.

Des weiteren ist es prinzipiell möglich, die Prothese nicht einstückig, sondern zweistückig, nämlich aus dem Platten- und dem Schaftteil bestehend, auszubilden. Es ist besonders vorteilhaft, die Oberfläche der Prothese zusätzlich mit einer aus pulverförmigem bioaktiven Material bestehenden Panierungsschicht zu versehen. Dabei hat es sich als zweckmässig erwiesen, dass diese Schicht aus homöologen Knochenpartikeln, beispielsweise Knochenspänen oder -pulvern, besteht.

Die Vorteile der erfindungsgemässen Gehörknöchelchen-Prothese bestehen vor allem darin, dass aufgrund der grundsätzlich anders orientierten Relativlage der Rille auf der Plattenoberseite in Verbindung mit der anatomisch gerechten Abwinkelung des Schief-Schaftes sowie der geometrischen Detailausbildung der Gesamtprothese, vor allem aber auch wegen der Auswahl des Prothesen-Materials erstmals eine Dauer-Prothese mit überragender lokaler, anatomiegerechter Plazierungsstabilität, mit ausgezeichneter Mikro-Hebelwirkung zur funktionsgerechten Schwingungsübertragung, mit universeller Verwendbarkeit in der restaurativen Chirugie des Mittelohrbereiches, mit mechanisch überaus einfacher «Trimmbarkeit» auf anatomische Individualerfordernisse sowie insbesondere mit ausgezeichneten Eigenschaften im komplizierten Gehörknöchelchen-Ketten-Trakt zur Verfügung gestellt werden kann.

In den Zeichnungen sind Ausführungsbeispiele der Erfindung schematisch dargestellt. Es zeigen:

Fig. 1 eine erste Ausführungsform der erfindungsgemässen Prothese (Schief-Schaft-Prothese) in Seitenansicht;

Fig. 2 die Vorderansicht der in Fig. 1 dargestellten Prothese;

Fig. 3 die Draufsicht der in Fig. 1 dargestellten Prothese;

Fig. 4 die Symmetrieverhältnisse der in Fig. 1 dargestellten Prothesenplatte;

Fig. 5 eine zweite Ausführungsform der erfindungsgemässen Prothese (Gerad-Schaft-Prothese) in Seitenansicht;

Fig. 6 die Vorderansicht der in Fig. 5 dargestellten Prothese;

Fig. 7 die Draufsicht der in Fig. 5 dargestellten Prothese;

Fig. 8 eine dritte, 2fach symmetrische Ausführungsform der Prothesenplatte;

Fig. 9 eine vierte, 1fach symmetrische Ausführungsform der Prothesenplatte;

Fig. 10 eine fünfte, 1fach symmetrische Ausführungsform der Prothesenplatte.

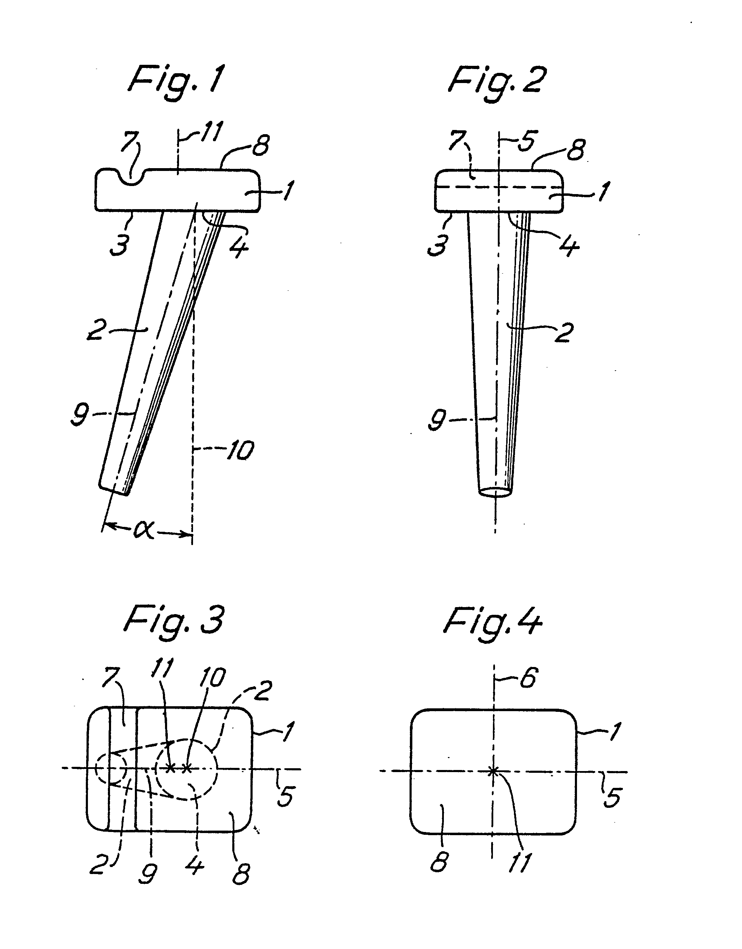

In Fig. 1 ist an eine planparallele Platte 1 ein im Querschnitt kreisförmiger Schaft angesetzt, dessen Achse 9 mit der auf der Plattenunterseite 3 stehenden Normalen 10 einen Winkel $\alpha$ bildet, der innerhalb eines Winkelintervalles von $10° \leqq \alpha \leqq 20°$ jeden beliebigen Wert annehmen kann.

Wie aus den Fig. 1 und 2 hervorgeht, verjüngt sich der Schaft 2 von der Plattenunterseite 3 zum Schaftende hin stetig. Die Platte 1 weist eine Rechteck-Form mit abgerundeten Ecken und Kanten auf. Sie besitzt eine auf der Zeichenebene senkrecht stehende Hauptsymmetrieebene, deren Spur 5 in den Fig. 2 bis 4 dargestellt ist. Eine Nebensymmetrieebene steht auf der Zeichenebene und auf der Hauptsymmetrieebene 5 der Platte senkrecht, vgl. die in Fig. 4 eingezeichnete Spur 6. Der Schnittpunkt beider Spuren 5 und 6 der genannten Symmetrieebene stellt den geometrischen Mittelpunkt 11 der Platte dar. Durch die Spur 5 der Hauptsymmetrieebene der Platte 1 und die Achse 9 des Schaftes 2 wird eine Ebene aufgespannt, die als Prothesen-Symmetrieebene bezeichnet wird. Ihre Spur fällt definitionsgemäss mit der Spur 5 zusammen.

Es ist aus Fig. 3 ersichtlich, dass die Ansatzfläche 4 des Schaftes 2 bzw. der Auftreffpunkt der Schaftachse 9 auf der Plattenunterseite 3 nicht mit dem Plattenmittelpunkt zusammenfällt. Der Schaft ist vielmehr exzentrisch an der Platte, und zwar an ihrem hinteren Teil, angesetzt. Wenn in der vorliegenden Anmeldung eine «Ansatzfläche 4» erwähnt und zeichnerisch dargestellt und wenn vom exzentrischen «Ansetzen» des Schaftes 2 an die Plattenunterseite 3 gesprochen wird, so geschieht dies lediglich zum Zwecke einer detaillierten, gegliederten Erläuterung der erfindungsgemässen Raumform. Die Prothese kann aus zwei Einzelstücken, nämlich aus der Platte 1 und dem Schaft 2 zusammengesetzt, sie kann aber ebenso einstückig ausgebildet sein.

Auf der Plattenoberseite 8 befindet sich senkrecht zur Symmetrieebene der Prothese eine Rille 7. Sie liegt im vorderen Teil der Plattenoberseite 8. Bezüglich des Plattenmittelpunktes 11 bzw. der Nebensymmetrieebene 6 kann auch von einer exzentrischen Positionierung der Rille 7 gesprochen werden. Es ist wesentlich, dass die Rille senkrecht zur Symmetrieebene der Prothese verläuft, weil auf diese Weise ein optimales formschlüssiges Eingreifen von noch vorhandenen Mittelohrtrakt-Fragmenten und damit eine verwacklungsfreie Langzeit-Positionierung des Implantates gewährleistet wird.

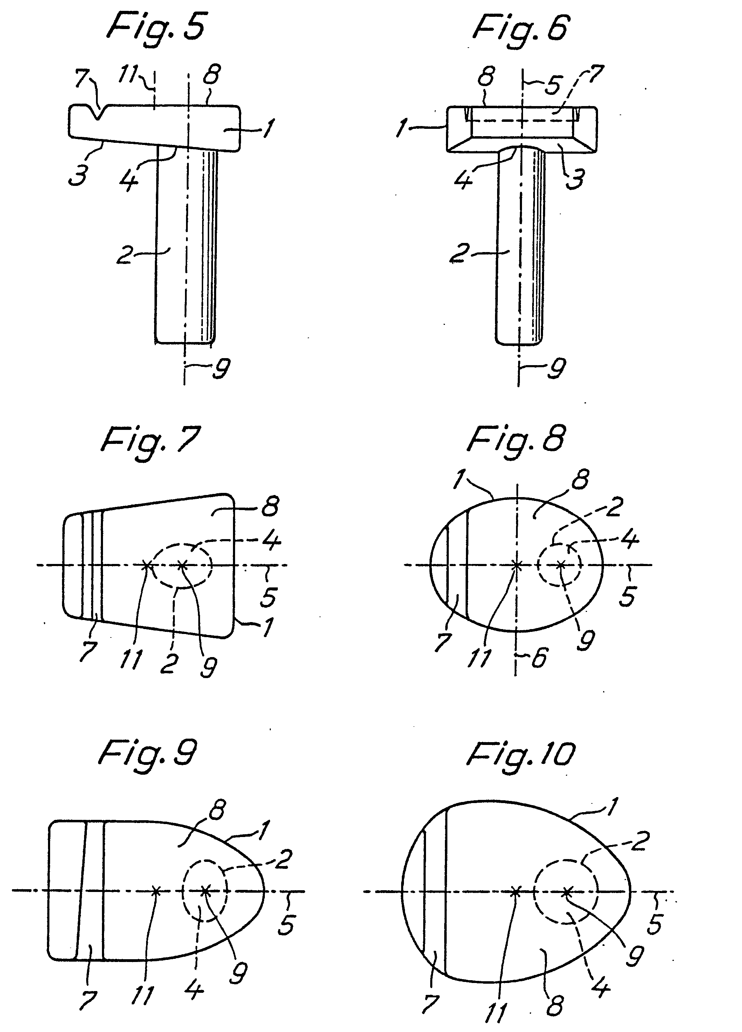

In den Figuren 5 bis 7 ist eine andere erfindungsgemässe Ausführungsform in drei verschiedenen Ansichten dargestellt. Die Platte 1 weist zwar eine plane Unterseite 3 und eine plane Oberseite 8 auf, jedoch nimmt die Plattendicke — ausgehend von ihrem Vorderteil zu ihrem Hinterteil — stetig zu.

Die Achse 9 des Schaftes 2 schliesst zwar mit der Normalen der Plattenunterseite 3 im dargestellten Fall einen kleinen Winkel ein, der der Neigung der Unterseite 3 gegenüber der horizontal verlaufenden Oberseite 8 entspricht; gleichwohl handelt es sich bei der in den Fig. 5 und 6 dargestellten Ausführungsform um eine «Gerad-Schaft-Prothese», da der Winkel zwischen der Plattenoberfläche 8 und der Schaftachse 9 ein Rechter ist. Der Schaft ist von elliptischem Querschnitt, wobei die Hauptachse der elliptischen Ansatzfläche 4 in der Spur 5 der Symmetrieebene der Prothese liegt. Es ist natürlich auch möglich, den elliptischen Schaftquerschnitt zu seinem Ende hin stetig zu verjüngen, wie es bei dem ersten Ausführungsbeispiel bereits dargestellt wurde. Auch liegt es im Rahmen der vorliegenden Erfindung, den Schaft bei Bedarf längs seiner Mantelfläche gezielt abzuflachen. Ebenso kann die Plattendicke im vorderen Bereich grösser sein als im hinteren Bereich. Des weiteren ist es möglich, die Rillenquerschnittsgeometrie den individuellen Erfordernissen gezielt anzupassen, vgl. Fig. 1 bzw. Fig. 5.

Eine Abstumpfung bzw. Abrundung aller Kanten und Ecken der Prothese, insbesondere der Plattenoberseite 8, ist wegen der enormen Perforationsbzw. Penetrations-Gefahr im Trommelfellbereich unbedingt erforderlich.

Die Platte 1 hat in Fig. 7 die Form eines Doppeltrapezes. Es ist indes auch möglich, andere Formen mit nur einer Symmetrieebene 5, wie sie etwa in Fig. 9 (schiffsbug-förmig) bzw. in Fig. 10 (birnenförmig) dargestellt sind, im Einzelfall vorzusehen oder aus einer auf Lager liegenden Prothese mit Rechteck-Plattenform zu fräsen. Eine höhersymmetrische Form mit Haupt- und Nebensymmetrieebene 5 bzw. 6 ist in Fig. 8 (ellipsenförmig) dargestellt. In allen Fällen liegt die Rille 7 im wesentlichen senkrecht zur Prothesensymmetrieebene 5.

In Fig. 9 ist eine Rillenform mit nichtparallelen Rillenkanten auf der Plattenoberfläche 8 dargestellt. Dies kann zu einer zusätzlichen Verkeilung und damit zu einem höheren Mass an mechanischer Verklammerung führen.

Allen vorgeschlagenen Detaillösungen sind jedoch die folgenden essentiellen Raumform-Merkmale gemeinsam: die Plattenform ist zumindest symmetrisch bezüglich der Symmetrieebene 5 der Gesamtprothese; die Ansatzfläche 4 des Schaftes 2 liegt immer exzentrisch bezüglich des Plattenmittelpunktes 11 sowie immer auf der Spur 5 der Symmetrieebene der Prothese, ohne jedoch selbst in den unmittelbaren Randbereich des hinteren Teils der Plattenunterseite 3 zu gelangen; die Rille liegt mindestens mit einer ihrer Kanten immer senkrecht zur Symmetrieebene 5 der Platte bzw. der Gesamtprothese und ausserdem immer exzentrisch bezüglich des Plattenmittelpunktes 11, ohne jedoch selbst in den unmittelbaren Randbereich des vorderen Teils der Plattenoberseite 8 zu gelangen.

Aus dieser Konstruktion resultiert eine optimale Mikro-Hebelwirkung, die zusammen mit einer mechanisch stabilen Gesamtkonstruktion sowie einer anatomisch passgenauen Lage am Implantationsort mitausschlaggebend für die überlegenen Eigenschaften der erfindungsgemässen Prothese sind.

Die positive Gesamtwirkung dieser neuartigen Prothesenform bei zahlreichen Applikanten wird noch durch die Auswahl an sich bekannter bioaktiver Materialien als Prothesenwerkstoff nachhaltig gesteigert. Obwohl der mit diesem Material prinzipiell erreichbare feste Implantat-Knochen-Verbund, wie er für die Verankerung von Knochen- und Gelenkersatz unbedingt benötigt wird, an dieser Stelle nicht gebraucht wird, hat sich die bioaktive Eigenschaft auch bei Implantaten für dieses Spezielgebiet der prothetischen Chirurgie besonders bewährt. Obwohl das Bio-Material nicht osteoinduktiv — also nicht primär knochenbildend — ist, kann etwa vorhandenes knöchernes Gewebe in der unmittelbaren Umgebung des Materials in Form von drei bis vier Zell-Lagen dicken Lamellen auf dem Bio-Material wachsen und das Implantat ummanteln.

Der Chirurg, der die erfindungsgemässe Gehörknöchelchen-Prothese zu implantieren beabsichtigt, kann sie unmittelbar vor dem Implantieren in vitro mit vom Patienten stammenden ( = antologen) feinsten Knochenpartikeln bestäuben oder die bereits in situ befindliche Prothese kann direkt während des Implantierungsvorganges mit feinstem Knochenpulver, das beim Anbringen geringfügiger Knochenwandkorrekturen oder bei sonstigen Mini-Fräs-, Bohr- und Schleifarbeiten im Mittelohrbereich anfällt, gleichsam gewollt beaufschlagt werden. Es hat sich aufgrund klinischer Tests überraschenderweise herausgestellt, dass sich bei derart locker bestreuten Gehörknöchelchen im Laufe eines einige Wochen dauernden kinetischen Bildungsprozesses in vivo eine lamellare Umhüllung auf ihrer Oberfläche bildet.

Es gilt als gesichert, dass dieser Bildungsvorgang in ursächlichem Zusammenhang mit dem ausgewählten bioaktiven Prothesen-Material steht.

**Patentansprüche**

1. Gehörknöchelchen-Prothese, enthaltend eine Platte (1) mit einer Plattenoberseite (8) und einer Plattenunterseite (3), wobei die Platte (1) eine spiegelsymmetrische Form bezüglich einer auf den durch die Plattenoberseite (8) und durch die Plattenunterseite definierten Ebenen senkrecht stehenden Symmetrieebene aufweist, einen an der Plattenunterseite (3) derart exzentrisch angesetzten Schaft (2), dass der geometrische Mittelpunkt von dessen Ansatzfläche (4) auf der Spur (5) dieser Symmetrieebene, jedoch mindestens um den entlang der Spur (5) gemessenen Halbmesser der Ansatzfläche (4) vom Rand der Platte (1) entfernt liegt, und eine die Plattenoberseite (8) durchlaufende Rille (7), dadurch gekennzeichnet, dass

a) die einzige Rille (7) auf der den geometrischen Mittelpunkt der Ansatzfläche (4) nicht enthaltenden Hälfte der Platte (1) angeordnet und senkrecht zu der Symmetrieebene ausgerichtet ist und

b) die Prothese aus bioaktivem Material besteht.

2. Gehörknöchelchen-Prothese nach Anspruch 1, dadurch gekennzeichnet, dass die Platte (1) die Form eines Rechteckes hat und so angeordnet ist, dass die längere Rechteckseite parallel zur Spur der Symmetrieebene (5) der Prothese verläuft.

3. Gehörknöchelchen-Prothese nach Anspruch 1, dadurch gekennzeichnet, dass die Platte (1) die Form einer Ellipse hat und so angeordnet ist, dass die längere Ellipsenachse mit der Spur der Symmetrieebene (5) der Prothese zusammenfällt.

4. Gehörknöchelchen-Prothese nach Anspruch 1, dadurch gekennzeichnet, dass die Achse (9) des Schaftes (2) mit der Normalen (10) der Plattenunterseite (3) einen Winkel $\alpha$ in der Grössenordnung von $\alpha = 15° \pm 5°$ einschliesst.

5. Gehörknöchelchen-Prothese nach einem der Ansprüche 1 bis 4, dadurch gekennzeichnet, dass das bioaktive Material ein Verbundmaterial ist.

6. Gehörknöchelchen-Prothese nach einem der Ansprüche 1 bis 4, dadurch gekennzeichnet, dass das bioaktive Material ein apatithaltiges Sinterprodukt ist.

7. Gehörknöchelchen-Prothese nach einem der Ansprüche 1 bis 4, dadurch gekennzeichnet, dass das bioaktive Material eine Glaskeramik ist.

8. Gehörknöchelchen-Prothese nach einem der Ansprüche 1 bis 4, dadurch gekennzeichnet, dass das bioaktive Material ein Glas ist. ·

9. Gehörknöchelchen-Prothese nach einem der Ansprüche 1 bis 8, dadurch gekennzeichnet, dass sie aus zwei Einzelteilen, nämlich Platte (1) und Schaft (2), zusammengesetzt ist.

## Claims

1. Prosthesis, for auditory ossicles, containing a plate (1) with an upper plate side (8) and a lower plate side (3), wherein the plate (1) displays a specularly symmetrical shape with respect to a plane of symmetry standing perpendicularly to the planes defined by the upper plate side (8) and the lower plate side, a shank (2) placed eccentrically against the lower plate side (3) in such a manner that the geometric centre of the connecting area (4) thereof lies on the track (5) of this plane of symmetry, however spaced from the rim of the plate (1) by at least half the diameter of the connecting area (4) measured along the track (5), and a groove (7) running through the upper plate side (8), characterised thereby, that

a) the single groove (7) is arranged on that half of the plate (1), which does not contain the geometric centre of the connecting area (4), and is aligned perpendicularly to the plane of symmetry and

b) the prosthesis consists of bio-active material.

2. Prosthesis, for auditory ossicles, according to claim 1, characterised thereby, that the plate (1) has the shape of a rectangle and is so arranged that the longer side of the rectangle extends parallelly to the track of the plane (5) of symmetry of the prosthesis.

3. Prosthesis, for auditory ossicles, according to claim 1, characterised thereby, that the plate (1) has the shape of an ellipse and is so arranged that the longer axis of the ellipse coincides with the track of the plane (5) of symmetry of the prosthesis.

4. Prosthesis, for auditory ossicles, according to claim 1, characterised thereby, that the axis (9) of the shank (2) includes an angle $\alpha$ in the order of magnitude of $\alpha = 15° \pm 5°$ with the normal (10) of the lower plate side (3).

5. Prosthesis, for auditory ossicles, according to one of the claims 1 to 4, characterised thereby, that the bio-active material is a compound material.

6. Prosthesis, for auditory ossicles, according to one of the claims 1 to 4, characterised thereby, that the bio-active material is a sintered product containing apatite.

7. Prosthesis, for auditory ossicles, according to one of the claims 1 to 4, characterised thereby, that the bio-active material is a glass ceramic material.

8. Prosthesis, for auditory ossicles, according to one of the claims 1 to 4, characterised thereby, that the bio-active material is a glass.

9. Prosthesis, for auditory ossicles, according to one of the claims 1 to 8, characterised thereby, that it is composed of two individual parts, namely plate (1) and shank (2).

## Revendications

1. Prothèse d'osselets de l'ouïe contenant une plaque (1) avec une face supérieure (8) de plaque et une face inférieure (3) de plaque, la plaque (1) présentant une forme spéculairement symétrique par rapport à un plan de symétrie placé perpendiculairement aux plans définis par la face supérieure (8) de plaque et par la face inférieure de plaque, avec un tige (2) rattachée excentriquement de telle façon à la face inférieure (3) de plaque que le centre géométrique de sa surface de raccordement (4) soit situé sur la trace (5) de ce plan de symétrie mais éloigné, du bord de la plaque (1), d'au moins le diamètre de la surface de raccordement (4) mesuré le long de la trace (5), et avec une rainure (7) traversant la face supérieure (8) de plaque, caractérisée en ce que

a) la rainure unique (7) est disposée sur la moitié de la plaque (1) ne contenant pas le centre géométrique de la surface de raccordement (4) et orientée perpendiculairement au plan de symétrie et

b) la prothèse est en matière bio-active.

2. Prothèse d'osselets de l'ouïe selon la revendication 1, caractérisée en ce que la plaque (1) a la forme d'un rectangle et est disposée de telle façon que le côté long du rectangle s'étende parallèlement à la trace du plan de symétrie (5) de la prothèse.

3. Prothèse d'osselets de l'ouïe selon la revendication 1, caractérisée en ce que la plaque (1) a la forme d'une éllipse et est disposée de telle façon que le grand axe de l'éllipse coïncide avec la trace du plan de symétrie (5) de la prothèse.

4. Prothèse d'osselets de l'ouïe selon la revendication 1, caractérisée en ce que l'axe (9) de la tige (2)

fait un angle $\alpha$ de l'ordre de grandeur de $\alpha = 15°\pm 5°$ avec la normale (10) à la face inférieure (3) de la plaque.

5. Prothèse d'osselets de l'ouïe selon l'une des revendications 1 à 4, caractérisée en ce que la matière bio-active est une substance composée.

6. Prothèse d'osselets de l'ouïe selon l'une des revendications 1 à 4, caractérisée en ce que la matière bio-active est un produit aggloméré contenant de l'apatite.

7. Prothèse d'osselets de l'ouïe selon l'une des revendications 1 à 4, caractérisée en ce que la matière bio-active est un vitrocérame.

8. Prothèse d'osselets de l'ouïe selon l'une des revendications 1 à 4, caractérisée en ce que la matière bio-active est un verre.

9. Prothèse d'osselets de l'ouïe selon l'une des revendications 1 à 8, caractérisée en ce qu'elle est composée de deux parties individuelles, à savoir la plaque (1) et la tige (2).

Fig. 1

Fig. 2

Fig. 3

Fig. 4

0 060 832

Fig.5

Fig.6

Fig.7

Fig.8

Fig.9

Fig.10

9